# EUROPÄISCHE PATENTANMELDUNG

(11) **EP 3 521 850 A1**
(43) Veröffentlichungstag der Anmeldung: **07.08.2019**
(21) Anmeldenummer: 18154727.4
(22) Anmeldetag: 01.02.2018
(51) Int. Cl.: G01R 33/54, G16H 40/60, A61B 5/055

(54) **BEREITSTELLEN VON BETRIEBSPARAMETERN VON MR-GERÄTEN**

(71) Anmelder: Siemens Healthcare GmbH, 91052 Erlangen (DE)
(72) Erfinder: Hammes, Martin, 91054 Erlangen (DE)

(57) **Zusammenfassung**

Die vorliegende Erfindung betrifft ein Verfahren und einen Ansatz zum Erzeugen einer Aggregationsdatei (A) auf einem MR-Scanner (MR). Es umfasst die Verfahrensschritte:
- Erfassen (S1) von Betriebsparametern (BP) auf dem MR-Scanner (MR);
- Aggregieren und Strukturieren (S3) der erfassten Betriebsparameter (BP) in ein vordefiniertes einheitliches Format zum Erzeugen (S4) der Aggregationsdatei (A).

Die Aggregationsdatei kann dazu verwendet werden, zu berechnen, welche klinische Fragestellung auf dem MR-Scanner beantwortet werden kann.

## Beschreibung

Die vorliegende Erfindung betrifft Methoden zum Bereitstellen von Betriebsparametern von MR-Geräten.

MR-Geräte (Magnetresonanztomographiegeräte) sind komplexe technische Maschinen, die eine Vielzahl von technischen Bauteilen umfassen. Sie dienen zur Ausführung von bildgebenden Verfahren zur medizinischen Diagnostik und werden zur Darstellung von Struktur und Funktion der Gewebe und Organe im Körper eingesetzt.

Das Verfahren beruht darauf, dass die Atomkerne im untersuchten Gewebe durch eine Kombination von statischen und hochfrequenten magnetischen Feldern gezielt phasensynchron zu einer bestimmten Bewegung angeregt werden und dann ein messbares Signal in Form einer Wechselspannung abgeben, bis die Bewegung abgeklungen ist. Dazu muss die Maschine mit einem Hauptmagneten und mehreren Gradientenspulen ausgebildet sein, um die Ortsauflösung des Signals erfassen zu können. Weiterhin ist ein Hochfrequenzsystem erforderlich, um die Spins durch ein kurzzeitig angelegtes zusätzliches hochfrequentes Wechselfeld anzuregen. Dieses besteht aus einem leistungsstarken Sender und einem extrem empfindlichen Empfänger. Nach Abschalten des hochfrequenten Wechselfeldes nimmt die transversale Magnetisierung (wieder) ab, die Spins richten sich also wieder parallel zum statischen Magnetfeld aus. Für diese sogenannte Relaxation benötigen sie eine charakteristische Abklingzeit. Diese ist von der chemischen Verbindung und der molekularen Umgebung abhängig, in der sich der präzedierende Wasserstoffkern befindet. Daher unterscheiden sich die verschiedenen Gewebearten charakteristisch in ihrem Signal, was zu verschiedenen Signalstärken (Helligkeiten) im resultierenden Bild führt.

Zur Erfassung des Signals der angeregten Kerne werden Antennen eingesetzt, in deren Lokalspule eine Spannung induziert wird, die dann (z.B. mit einem rauscharmen Vorverstärker, LNA) verstärkt und schließlich kabelgebunden an die Empfangselektronik weitergeleitet wird. Zur weiteren Datenerfassung können zusätzliche Bauteile eingesetzt werden. So können z.B. an ein MRT-Empfangssystem mehr Spulenelemente (Loops) angeschlossen werden, als Empfänger vorhanden sind. In diesem Fall wird zwischen Empfangsantennen und Empfänger eine Schaltmatrix eingebaut. Diese routet die momentan aktiven Empfangskanäle auf die vorhandenen Empfänger. Dadurch ist es möglich, mehr Spulenelemente anzuschließen, als Empfänger vorhanden sind, da bei einer Ganzkörperabdeckung nur die Spulen ausgelesen werden müssen, die sich im FoV (Field of View) bzw. im Homogenitätsvolumen des Magneten befinden.

Die im Einsatz befindlichen MR-Geräte unterscheiden sich hinsichtlich ihres Aufbaus und den darauf ausführbaren Funktionen. So können z.B. je nach Positionierung und Anzahl der Gradientenspulen unterschiedliche anatomische Körperbereichen und damit unterschiedliche, medizinische Fragestellungen adressiert werden. Weiterhin beeinflussen z.B. die Stärke des Hauptmagneten, die erzeugte Homogenität des erzeugten Magnetfeldes, die Kanalanzahl und der FOV-Bereich die möglichen Konfigurationen für ein Messprotokoll zur Patientenuntersuchung.

Für die Praxis ist es somit wichtig, Information über die technische Ausstattung und Konfiguration eines MR-Gerätes z.B. mit den oben erwähnten Bauteilen (B0-Spule, Gradientenspulen, HF-Antennen, Kanäle etc.) zu erhalten, die die verfügbaren Fähigkeiten des MR-Gerätes repräsentieren und diese interoperabel zur Verfügung zu stellen, um z.B. den Einsatz des MR-Gerätes verbessert planen zu können.

Bei den im Stand der Technik eingesetzten MR-Systemen ist es nicht möglich, diese Informationen direkt aus den Geräten automatisiert auszulesen. Das heißt, klinisches Personal (Radiologen, MTRAs) müssen wissen, welche Fähigkeiten ihr Gerät hat, um dann die Patienten entsprechend zu planen. Dieses individuelle und manuelle Vorgehen erweist sich insbesondere dann als schwierig und fehleranfällig, wenn mehrere MR-Geräte mit unterschiedlicher Ausstattung gemanaged werden müssen (bspw. bei Krankenhausketten oder Großkrankenhäusern). Ein Geräte-Scheduling erfordert somit nachteiligerweise die Erfahrung der Mitarbeiter.

Der vorliegenden Erfindung liegt die Aufgabe zugrunde, das Betreiben eines medizintechnischen Gerätes, insbesondere eines MR-Gerätes, im klinischen Einsatz zu verbessern. Insbesondere soll die technische Planung des MR-Gerätes automatisiert werden. Dafür sollen die erforderlichen Informationen mit den Betriebsparametern des MR-Scanners verbessert und interoperabel bereitgestellt werden.

Diese Aufgabe wird jeweils durch einen Gegenstand nach den unabhängigen, einander nebengeordneten Ansprüchen gelöst. Vorteilhafte Ausführungsformen sind Gegenstand der abhängigen Ansprüche, der Beschreibung und der Zeichnungen.

Im Folgenden wird die Lösung der Aufgabe unter Bezugnahme auf einen MR-Scanner (bzw. MR-Gerät) beschrieben. Für einen Fachmann liegt es jedoch auf der Hand, dass die hier vorgeschlagene Lösung auch auf andere medizintechnische Geräte oder Modalitäten (CT, US, PET etc.) angewendet werden kann. Die Betriebsparameter spezifizieren dann die technischen Eigenschaften und Konfigurationen des jeweiligen Gerätes.

Gemäß einem ersten Aspekt wird die Aufgabe durch ein Verfahren zum Erzeugen einer Aggregationsdatei auf einem MR-Scanner gelöst. Das Verfahren gliedert sich in zwei Verfahrensschritte:
- Erfassen von Betriebsparametern, die verfügbare technische Eigenschaften und/oder Funktionalitätsdaten auf dem MR-Scanner repräsentieren; dazu können vorzugsweise Sensordaten und Daten von im MR-Scanner verbauten Bauteilen erfasst und verarbeitet werden. Die Sensordaten können z.B. von Sensoren in bestimmten Bauteilen von dem MR-Scanner stammen, die z.B. die Position und Anzahl der Gradientenspulen indizieren. Aus diesen Sensordaten und weiteren Daten können mittels eines Detektionsalgorithmus' Betriebsparameter ermittelt werden. Andere Sensoren können z.B. zur Ermittlung der Magnetfeldstärke dienen, aus denen der Detektionsalgorithmus mit oder ohne Zugriff auf eine Datenbank mit Zuordnungsrelationen, weitere Betriebsparameter ermittelt werden können.
- Aggregieren und Strukturieren der erfassten Betriebsparameter in ein vordefiniertes, einheitliches Format zum Erzeugen der Aggregationsdatei.

Das Verfahren hat den technischen vorteilhaften Effekt, dass die Aggregationsdatei ein vorgegebenes, einheitlich strukturiertes Format aufweist. Damit wird die Interoperabilität zwischen den MR-Geräten verbessert oder überhaupt hergestellt (z.B. bei Geräten von unterschiedlichen Herstellern). Insbesondere können auch lokale und/oder hersteller-spezifische Betriebsparameter einfach und unkompliziert ohne weitere Zwischeninstanzen (z.B. zur Konvertierung) ausgetauscht werden. Externe Systeme und hersteller-fremde weitere MR-Geräte können nun auf automatisierte Weise die notwendigen Daten in Form der Aggregationsdatei entweder aktiv abrufen (PULL-Betrieb) oder passiv empfangen (PUSH-Betrieb), z.B. mit Implementierung einer Pollingstrategie mit zyklischen Abfragen nach geänderten oder neuen Betriebsparametern oder Änderungen an der Aggregationsdatei, um diese zu verarbeiten.

In einer allgemeinen Formulierung betrifft die Erfindung ein einheitliches Austauschformat für geräteinterne Betriebsparameter von medizinischen Geräten. Das Austauschformat wird mittels Vorgaben zum Erzeugen einer Aggregationsdatei definiert. Diese Vorgaben werden von einem Strukturierungsalgorithmus eingelesen und umgesetzt, um die Aggregationsdatei zu erzeugen.

Das Aggregieren und Strukturieren der erfassten Betriebsparameter in das einheitliche Format (der Aggregationsdatei) wird somit vorzugsweise mittels des Strukturierungsalgorithmus ausgeführt. Der Strukturierungsalgorithmus kann Vorgabedaten für das zu erzeugende Format aus einer externen Datenbank einlesen. Dies hat den Vorteil, dass das Format auch unabhängig vom Algorithmus, der auf einer Recheneinheit auf dem MR-Scanner implementiert sein kann, geändert werden kann.

In einer vorteilhaften Ausführungsform der Erfindung umfassen die Betriebsparameter eine Anzahl der verfügbaren Spulen, einen Typ der verfügbaren Spulen, ausführbare Protokolle, eine Magnetfeldstärke, eine Gradientenfeldstärke, eine Feldhomogenität, eine Kanalzahl und/oder einen Bildbereich (field of view - FOV). Die Betriebsparameter können sich auf Hardwareparameter des MR-Gerätes und/oder auf Softwareparameter beziehen, wie z.B. verfügbare, installierte Software-Lizenzen. Damit können vorteilhafterweise alle relevanten technischen Eigenschaften des Systems repräsentiert und erfasst werden.

Gemäß einer weiteren, vorteilhaften Ausführungsform der Erfindung wird mittels eines Induktionsalgorithmus' aus der erzeugten Aggregationsdatei und/oder direkt aus den erfassten Betriebsparametern automatisch berechnet, welche klinische Fragestellung auf dem MR-Scanner beantwortet werden kann. Der Induktionsalgorithmus kann zur Berechnung auf eine Datenbank zugreifen, in der Zuordnungen zwischen jeweils einer klinischen Fragestellung und den dafür erforderlichen technischen Gerätekonfigurationen (die in den Betriebsparametern codiert sind) abgelegt sind. Dies vereinfacht den Betrieb des MR-Scanners deutlich, da diese wichtige Information somit automatisch und nicht mehr durch den Einsatz von Fachpersonal bereitgestellt werden kann.

In einer weiteren, vorteilhaften Ausführungsform der Erfindung wird mittels eines Scanzeitalgorithmus' aus der erzeugten Aggregationsdatei und/oder direkt aus den erfassten Betriebsparametern bei einer gegebenen klinischen Fragestellung automatisch prognostiziert werden, wie lange eine durchschnittliche Scanzeit (Untersuchungsdauer) auf Basis von auszuführenden Protokollen und Messsequenzen sein wird. Damit kann die erzeugte Aggregationsdatei genutzt werden, um ein Scheduling des Geräte-Einsatzes zu automatisieren und verbessert auszuführen.

In einer weiteren, vorteilhaften Ausführungsform der Erfindung werden die Betriebsparameter aus lokalen Gerätedaten berechnet. Dabei kann es sich z.B. um die Zeitdauer der Untersuchung (Scanzeit) handeln, die auf Basis von verfügbaren Protokollen berechnet wird. Unter Zugriff auf weitere Planungsdaten (z.B. Vorbereitungszeit eines Patienten) und der berechneten Scanzeit, kann eine verbesserte Planung und Auslastung des MR-Scanners erzielt werden. Es ist auch möglich, die Vorbereitungszeit zu messen, z.B. als Zeitspanne zwischen Registrierung des Patienten und Beginn des Scans. Somit kann das Scheduling verbessert werden. Die errechneten und/oder gemessenen Daten (z.B. Vorbereitungszeit, slot time) können in der Aggregationsdatei zur Verfügung gestellt werden, was die Planung eines Systems von mehreren MR-Scannern (z.B. in einer Klinik) deutlich vereinfacht und verbessert. Insgesamt kann damit die Effizienz des MR-Gerätebetriebs erhöht werden.

Gemäß einer weiteren, vorteilhaften Ausführungsform der Erfindung kann eine Zuordnung zwischen jeweils einer beantwortbaren klinischen Fragestellung und einer Menge von dafür "erforderlichen" Betriebsparametern des MR-Scanners gespeichert werden. Dies kann zentral in einer Datenbank erfolgen. Dies bringt den technischen Vorteil, dass die Beantwortbarkeit der klinischen Fragestellungen automatisiert werden kann, indem die Information auf einfache Weise aus der erzeugen Aggregationsdatei ausgelesen wird.

Gemäß einer weiteren, vorteilhaften Ausführungsform der Erfindung werden Zuordnungen zwischen jeweils einer beantwortbaren klinischen Fragestellung und einer Menge von "erforderlichen" Betriebsparametern des MR-Scanners durch Verfahren des maschinellen Lernens gelernt werden. Dazu kann z.B. ein künstliches neuronales Netz (ANN - artificial neural network) eingesetzt werden, das in einer Trainingsphase mit Trainingsdaten trainiert worden ist. Das ANN kann mehrere Schichten umfassen. Maschinelles Lernen ist ein selbstadaptiver Algorithmus. Deep Learning, eine Teilmenge des maschinellen Lernens, nutzt eine Reihe hierarchischer Schichten bzw. eine Hierarchie von Konzepten, um den Prozess des maschinellen Lernens durchzuführen. Die hierbei benutzten künstlichen neuronalen Netze umfassen Strukturen, auch "Neuronen" genannt, die, wie ein Netz, miteinander verbunden sind. Die erste Schicht des neuronalen Netzes, der sichtbare "input Layer", verarbeitet eine Rohdateneingabe, wie z. B. die erfassten Betriebsparameter des MR-Gerätes. Die Dateneingabe enthält Variablen, die beobachtet werden können, daher "sichtbare Schicht". Diese erste Schicht leitet ihre Ausgaben an die nächste Schicht weiter. Diese zweite Schicht verarbeitet die Informationen der vorherigen Schicht und gibt das Ergebnis ebenfalls an innere Schichten ("hidden layers") weiter. Das Ergebnis wird im sichtbaren "output layer", der letzten Schicht, ausgegeben. Hierdurch wird die gewünschte Datenverarbeitung (hier: das Lernen von Zuordnungen zwischen erfassten Betriebsparametern, daraus berechneten weiteren Betriebsparametern und damit beantwortbaren klinischen Fragestellungen) in eine Reihe von verschachtelten einfachen Zuordnungen unterteilt. Ein solches ANN kann auf dem Prozessor implementiert sein. Das ANN kann zur Vorhersage von Untersuchungsdaten für das MR-Gerät verwendet werden. So kann das ANN z.B. trainiert werden, um einen Forecast der Scandauer und ggf. der Atemanhaltezeit bereitzustellen auf Basis der erfassten Betriebsparameter.

Gemäß einer weiteren, vorteilhaften Ausführungsform der Erfindung umfasst der Schritt des Aggregierens und Strukturierens ein Konvertieren der erfassten Betriebsparameter. Dies hat den Vorteil, dass die Aggregationsdatei herstellerunabhängig und somit unabhängig von einem spezifischen Format bereitgestellt werden kann. Dies verbessert die Interoperabilität des Verfahrens.

In einer anderen vorteilhaften Weiterbildung der Erfindung werden die Betriebsparameter auf Basis von Sensordaten und/oder aus elektronischen Daten des MR-Scanners und seiner Bauteile mittels eines Detektionsalgorithmus' ermittelt. Die Sensoren können von unterschiedlichem Typ sein und z.B. über Lagesensoren die Verbau-Situation (z.B. von Spulen) und die Konfiguration des Gerätes erfassen. Die elektronischen Daten sind lokal auf dem MR-Gerät verfügbar (lokal gespeichert) und/oder können über Netzwerkschnittstellen von externen Instanzen (z.B. PACS, RIS) eingelesen werden. Die elektronischen Daten können z.B. über ein internes Bus- oder Kommunikationssystem aus den Bauteilen des Scanners ausgelesen werden.

Gemäß einer weiteren, vorteilhaften Ausführungsform der Erfindung wird die Aggregationsdatei lokal auf dem MR-Scanner gespeichert. Sie kann an externe Instanzen übertragen werden. Dabei sind zwei Ausführungsvarianten der Erfindung vorgesehen. Eine erste Lösung ist, dass der Scanner die erzeugte Aggregationsdatei - z.B. per Email oder mit einer PUSH-Nachricht - aktiv gemäß einem Push-System verschickt. In einer zweiten Lösung wird eine PULL-Logik verwendet. Diese hat den Vorteil, dass an dem Scanner selbst (der als Sender der erzeugten Aggregationsdatei fungiert) die Verteilerliste der Empfänger nicht administriert werden muss. Dies bedeutet, dass sich die anfragenden Systeme die Aggregationsdatei holen und diese somit auf dem Sendeknoten passiv auf Anfrage bereitgestellt wird. Bei Änderungen am System werden die entsprechenden Informationen per PUSH verteilt.

Eine weitere Aufgabenlösung sieht ein Computerprogramm vor, mit Computerprogrammcode zur Durchführung aller Verfahrensschritte des oben näher beschriebenen Verfahrens, wenn das Computerprogramm auf einem Computer ausgeführt wird. Dabei ist es auch möglich, dass das Computerprogramm auf einem von einem Computer lesbaren Medium gespeichert ist.

Vorstehend wurde die Lösung der Aufgabe anhand des Verfahrens beschriebenen. Dabei erwähnte Merkmale, Vorteile oder alternative Ausführungsformen sind ebenso auch auf die anderen beanspruchten Gegenstände zu übertragen und umgekehrt. Mit anderen Worten können auch die gegenständlichen Ansprüche (die beispielsweise auf ein MR-System oder auf ein Computerprogrammprodukt gerichtet sind) mit den Merkmalen weitergebildet sein, die in Zusammenhang mit dem Verfahren beschrieben oder beansprucht sind. Die entsprechenden funktionalen Merkmale des Verfahrens werden dabei durch entsprechende gegenständliche Module, insbesondere durch Hardware-Module oder Mikroprozessor-Module, des Systems bzw. des Produktes ausgebildet und umgekehrt.

In einem weiteren Aspekt bezieht sich die Erfindung auf ein MR-basiertes System mit zumindest einem MR-Scanner und zumindest einer elektronischen Instanz, die zum Verarbeiten von Betriebsparametern des MR-Scanners ausgebildet ist und wobei die Betriebsparameter in einer Aggregationsdatei bereitgestellt werden, die nach einer der oben beschriebenen Ausgestaltungen des Verfahrens erzeugt worden ist.

In einem weiteren Aspekt bezieht sich die Erfindung auf Computerprogrammprodukt (das z.B. als Speichermedium oder als computerlesbares Medium ausgebildet sein kann), das Betriebsparameter in einer Aggregationsdatei speichert, die gemäß einem Verfahren mit den Schritten nach einem der vorangehenden Verfahrensansprüche erzeugt wurde. Zudem können die Vorgaben und Befehle zur Erzeugung der Aggregationsdatei in dem Computerprogrammprodukt gespeichert sein. Sobald also das Computerprogrammprodukt auf einem MR-Gerät installiert ist, kann die Funktionalität zur Erzeugung der Aggregationsdatei bereitgestellt werden.

Im Folgenden werden die in dieser Anmeldung verwendeten Begrifflichkeiten näher erläutert.

Mit dem Begriff 'Betriebsparameter' sind die technischen oder physikalischen Fähigkeiten des MR-Gerätes zusammengefasst. Dies umfasst die technische Konfiguration des Gerätes und insbesondere Angaben, wie lange eine bestimmte Akquisition auf einem bestimmten MR Gerät dauert, welcher Aufwand (bspw. für ein Shimming oder die Patientenpositionierung) benötigt wird, welche Lizenzen und Spulen vorhanden sind, um überhaupt bestimmte Scans durchführen zu können, etc. Die Betriebsparameter repräsentieren die technischen Eigenschaften des MR-Systems und können auch Funktionalitätsdaten (welche Funktionen können auf dem Gerät ausgeführt werden) umfassen. Die Betriebsparameter sind in der Regel lokal auf dem Gerät vorhanden. Sie können aus einer Datei eingelesen werden. Die Betriebsparameter können auf Basis von Sensordaten, die von Sensoren in den jeweiligen Bauteilen des Gerätes stammen, erfasst werden.

Aggregieren bedeutet ein koordiniertes Zusammenfassen der erfassten Betriebsparameter. Da die Betriebsparameter an unterschiedlichen Stellen im Gerät anfallen, müssen diese zunächst an einer zentralen Stelle (z.B. von allen Sensoren mit der jeweils zugeordneten Datenverarbeitungseinheit) gesammelt werden.

Das Format bezieht sich auf die logische Struktur der Aggregationsdatei und insbesondere auf eine Abfolge der Einträge der Betriebsparameter. Dabei kann vorzugsweise ein einheitliches Format für die Art der Betriebsparameter definiert werden (Einheit, Format des Parameters, z.B. eine Zeitangabe in Sekunden oder Millisekunden etc.). Herstellerspezifische oder geräte-spezifische Angaben werden vorzugsweise in ein herstellerunabhängiges und geräteunabhängiges Format formatiert. Die Vorgaben für die logische Struktur der Aggregationsdatei können als Standard festgelegt werden, um den Datenaustausch zwischen unterschiedlichen Systemen zu ermöglichen oder vereinfachen. Der Standard oder die Vorgaben für das Format können in einer zentralen Datenbank hinterlegt sein.

Eine "klinische Fragestellung" ist ein elektronischer Datensatz, der repräsentiert, welche klinische Untersuchung durchgeführt werden soll, also bspw. was in welcher Körperregion untersucht werden kann und für welche Art von Patienten (z.B. Erwachsene, Kinder, Personen mit aktiven/passiven Implantaten, Schwangere, etc.) eine entsprechende Untersuchung durchgeführt werden kann. Fragestellungen im Kopf sind beispielsweise: Schlaganfall, Verdacht auf Tumor, Multiple Sklerose, Alzheimer etc. Je nach Funktionalität des MR-Scanners und dessen Konfiguration und den installierten Bauteilen, können unterschiedliche klinische Fragestellungen beantwortet werden. Für jede klinische Fragestellung muss ein ausführbares Protokoll und die dafür erforderlichen Bauteile (verfügbare Spulen etc.) zur Durchführung des Protokolls hinterlegt sein, sowie die Information, ob für bestimmte Patienten die klinische Fragestellung nicht beantwortet werden kann (bspw. aus Sicherheitsgründen für Implantate). Aus den Protokollinformationen muss dann die Zeitdauer und beispielsweise die maximale Atemanhaltezeit ausgelesen werden. Die Zuordnung des Protokolls zur klinischen Fragestellung kann entweder durch den Hersteller bei Auslieferung des Systems erfolgen oder auch seitens des Kunden. Die Protokolle werden wiederum davon beeinflusst welche MR-Sequenzen, Spulen und weitere Sensoren (bspw. Atemsensoren, Cardiac Triggering Sensoren) zur Verfügung stehen und im Protokoll verwendet werden. Dies wird mit Sensoren erfasst. Außerdem beeinflussen die HW-Basischarakteristika des Scanners (Feldstärke, Feldhomogenität, FOV, Kanalanzahl etc.) die Konfiguration des Protokolls.

In der folgenden detaillierten Figurenbeschreibung werden nicht einschränkend zu verstehende Ausführungsbeispiele mit deren Merkmalen und weiteren Vorteilen anhand der Zeichnung besprochen. In dieser zeigen:
- Fig. 1: eine schematische Darstellung eines MR-Systems, das zum Erzeugen einer Aggregationsdatei ausgebildet ist;
- Fig. 2: ein Blockschaltbild eines MR-Gerätes zum Erzeugen einer Aggregationsdatei gemäß einer bevorzugten Ausführungsform der Erfindung;
- Fig. 3: ein Ablaufdiagramm eines Verfahrens gemäß einer bevorzugten Ausführungsform der Erfindung;
- Fig. 4: ein System von mehreren medizinischen Geräten, die Aggregationsdateien erzeugen und austauschen und

- Fig. 5: eine schematische Darstellung eines Prozessors zum Erzeugen einer Aggregationsdatei gemäß einer weiteren bevorzugten Ausführungsform der Erfindung.

Im Folgenden wird die Erfindung unter Bezugnahme auf die Figuren und unterschiedlichen Ausführungsformen der Erfindung näher erläutert.

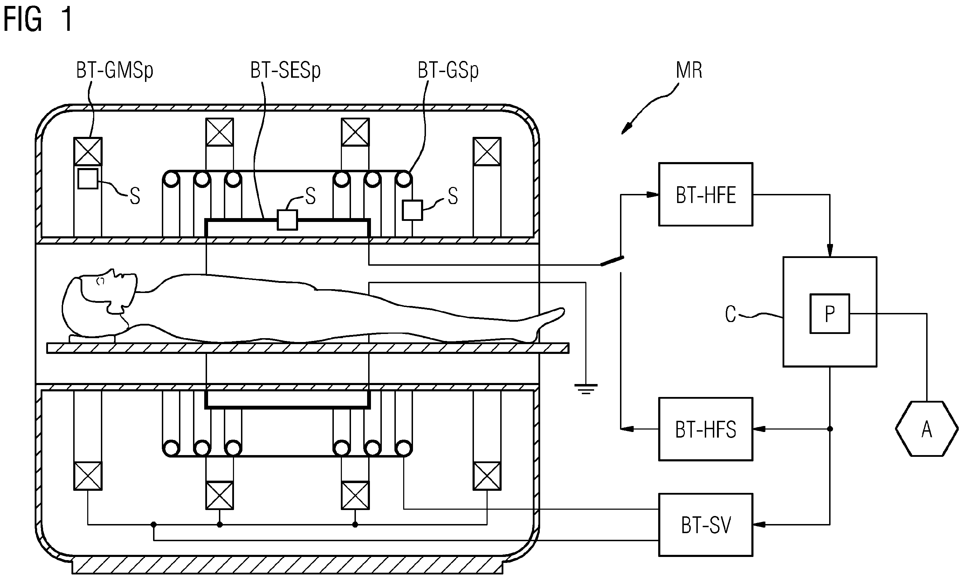

Fig. 1 zeigt eine schematische Übersichtsdarstellung eines MR-Gerätes MR mit unterschiedlichen Bauteilen BT, umfassend eine Grundmagnetfeldspule BT-GMSp, mehrere Gradientenspulen BT-GSp zur Ortsauflösung, und HF-Sende- und Empfangsspulen BT-SESp sowie ein HF-Empfängerbauteil BT-HFE und ein HF-Sendebauteil BT-HFS. Dies ist nur eine schematische Darstellung, so dass dem Fachmann offensichtlich ist, dass noch weitere (in Fig. 1 nicht gezeigte) Bauteile BT, wie z.B. Shimspulen zur Homogenisierung der Magnetfelder etc. eingesetzt werden. Wie in Fig. 1 ersichtlich, können an allen oder ausgewählten Bauteilen BT Sensoren S angeordnet sein, um die Existenz, Position, Ausprägung (z.B. Typ), Anordnung und/oder weiteren technischen Eigenschaften des jeweiligen Bauteils BT zu erfassen. An den MR-Scanner MR ist über eine entsprechende Datenverbindung ein Hochleistungsrechner als Computereinheit C angeschlossen.

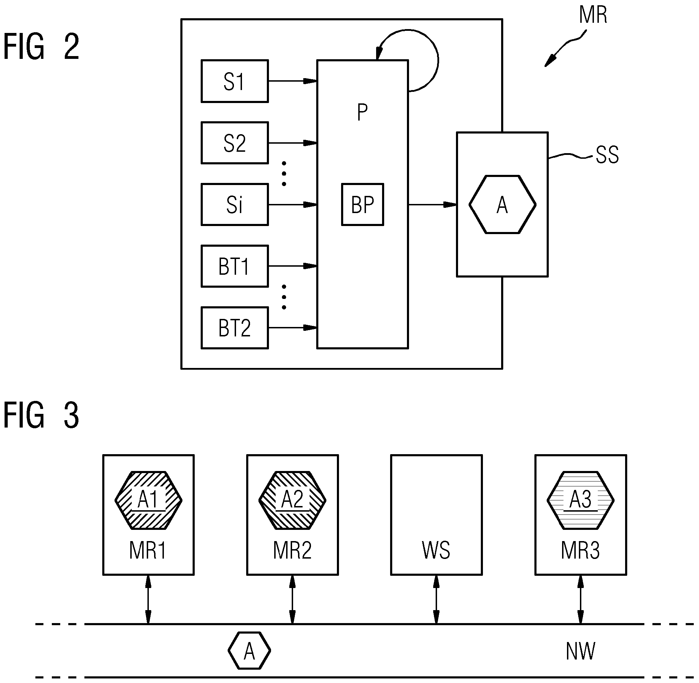

Fig. 2 zeigt den MR-Scanner MR mit weiteren Modulen. Auf dem MR-Scanner MR ist ein Prozessor P installiert, der als Recheneinheit fungiert. Die vorstehend genannten Sensoren S1, S2,...Si liefern die erfassten Sensordaten an den Prozessor P. Die Sensordaten können z.B. die Position und Art einer Spule repräsentieren. Aus den erfassten Sensordaten kann der Prozessor P direkt oder mittels eines Detektionsalgorithmus die Betriebsparameter BP ermitteln oder berechnen. Weiterhin empfängt der Prozessor P Daten von den Bauteilen BT direkt. Der Prozessor P kann mittels des Detektionsalgorithmus aus den erfassten Daten weitere Betriebsparameter BP berechnen. Dies soll in Fig. 2 mit dem Pfeil gekennzeichnet sein, der von dem Prozessor P auf sich selbst gerichtet ist. Es werden also aus den erfassten und/oder bereits vorliegenden Daten weitere Betriebsparameter BP berechnet, um all die gesammelten Daten als Betriebsparameter BP zu aggregieren. Die Betriebsparameter repräsentieren vorzugsweise Angaben zu: Verfügbaren Lokalspulen, verfügbaren Softwarelizenzen auf dem Computer C, Scannerbasisfunktionalitäten (bspw. Feldstärke, Gradientenstärke etc.). Die derart aggregierten Betriebsparameter BP werden gemäß einem vorgegebenen Format strukturiert und in diesem einheitlich strukturierten Format zum Erzeugen einer Aggregationsdatei A verwendet. Die Aggregationsdatei A kann über eine Netzwerk-Schnittstelle SS an externe Instanzen weitergeleitet werden. Aufgrund der Tatsache, dass die Aggregationsdatei A ein einheitlich strukturiertes Format aufweist, wird die Interoperabilität zwischen unterschiedlichen MR-Geräten MR gewährleistet, die bei Implementierung dieser Erfindung vorteilhafterweise auch von unterschiedlichen Herstellern stammen können.

Fig. 3 zeigt ein MR-System mit mehreren MR-Geräten MR1, MR2, MR3 und einer mit dem Bezugszeichen WS gekennzeichneten Workstation. Jedes MR-Gerät MR1, MR2, MR3 erzeugt eine geräte-spezifische Aggregationsdatei A1, A2, A3, die über das Netzwerk NW kommuniziert und z.B. von der externen Workstation WS eingelesen werden kann (PULL- oder PUSH-Betrieb). In Fig. 3 soll die Form der Aggregationsdatei A das einheitliche Format derselben kennzeichnen. Jede Aggregationsdatei A ist als 6-Eck dargestellt. Dies soll repräsentieren, dass das Format aller Aggregationsdateien A einheitlich ist. Die in der Aggregationsdatei A gespeicherten Daten sind jedoch MR-Gerätespezifisch und Konfigurationsspezifisch und unterscheiden sich von Datei zu Datei. Der unterschiedliche Dateninhalt soll in Fig. 3 mit der unterschiedlichen Schraffur gekennzeichnet sein bei gleicher Form (Format) der Aggregationsdatei A.

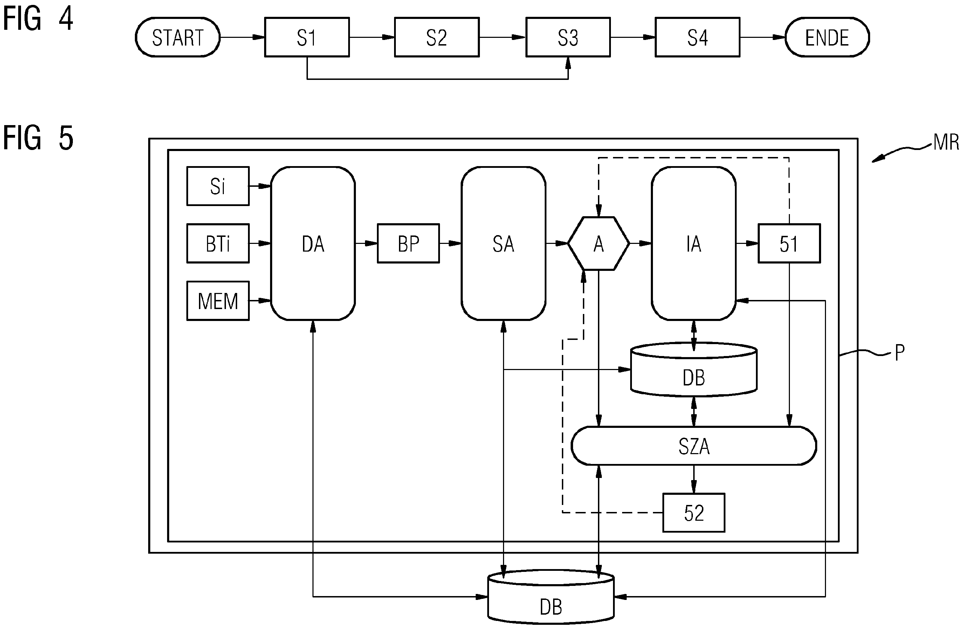

Fig. 4 ist ein Ablaufdiagramm des erfindungsgemäßen Verfahrens. Nach dem Start des Verfahrens zum Erzeugen der Aggregationsdatei A auf dem MR-Scanner MR werden in Schritt S1 die Betriebsparameter BP mit den verfügbaren Eigenschaften und/oder Funktionalitätsdaten auf dem MR-Scanner MR erfasst. Dazu können Sensordaten eingelesen werden, die auf Sensoren S erfasst worden sind. Die Sensoren S sind auf den Bauteilen BT des MR-Gerätes MR angeordnet, die relevant für die Betriebsparameter und insbesondere relevant für die Frage sind, welche klinischen Fragestellungen auf dem Gerät MR beantwortet werden können. In Schritt S2 werden aus den erfassten und berechneten Daten weitere Betriebsparameter BP berechnet. Der Schritt S2 ist jedoch optional, falls ein umfassenderes Bild in der Aggregationsdatei A erfasst werden soll. In Schritt S3 werden die erfassten und berechneten Betriebsparameter BP in ein vordefiniertes, einheitliches Format aggregiert und strukturiert, um nachfolgend in Schritt S4 die Aggregationsdatei A zu erzeugen. Danach kann das Verfahren wiederholt ausgeführt werden oder enden.

Fig. 5 zeigt ein MR-Gerät MR mit dem Prozessor P. Auf dem Prozessor P ist zumindest ein Detektionsalgorithmus DA implementiert, der in diesem Ausführungsbeispiel der Erfindung dazu ausgebildet ist, Betriebsparametern BP bereitzustellen (direkt zu erfassen und/oder zu berechnen). Auf dem Prozessor P ist zumindest ein weiterer Algorithmus, der Strukturierungsalgorithmus SA implementiert. Der Strukturierungsalgorithmus SA dient zur Strukturierung der aggregierten Betriebsparameter BP nach vordefinierten Vorgaben, insbesondere in einem einheitlichen Format. Dazu kann er auf Vorgaben zugreifen, die in einer zentralen Datenbank DB abgelegt sind. Der Strukturierungsalgorithmus SA erzeugt die Aggregationsdatei A.

In einer einfachen Ausführungsform der Erfindung sind die möglichen weiteren, im Folgenden beschriebenen Algorithmen nicht auf dem Prozessor P implementiert, sondern auf externen computerbasierten Instanzen, die über mehr Rechenleistung verfügen.

In einer komplexeren Ausführungsform der Erfindung, die in Fig. 5 dargestellt ist, kann auf dem Prozessor P zusätzlich ein Indikationsalgorithmus IA und/oder ein Scanzeit-Algorithmus SZA implementiert sein. Der Indikationsalgorithmus IA dient zur Berechnung der beantwortbaren klinischen Fragestellung, die in Fig. 5 mit dem Bezugszeichen 51 gekennzeichnet ist. Der Indikationsalgorithmus IA kann zur Berechnung auf die Datenbank DB zugreifen, um zur Berechnung notwendige Daten zu erfragen. Die berechneten, beantwortbaren klinischen Fragestellungen 51 können in der Aggregationsdatei A gespeichert werden (in Fig. 5 gestrichelt dargestellt).

Der Scanzeit-Algorithmus SZA dient zur Berechnung einer Scanzeit, die in Fig. 5 mit dem Bezugszeichen 52 gekennzeichnet ist. Die berechnete Scanzeit 52 kann ebenfalls in die Aggregationsdatei zurückgespeichert werden (gestrichelte Linie in Fig. 5).

Die Datenbank DB ist vorzugsweise außerhalb des MR-Scanners MR angeordnet und zentral für alle MR-Scanner des Systems.

In einer bevorzugten Ausführungsform der Erfindung ist der Prozessor P ausgebildet, aus der Aggregationsdatei A folgende Datensätze zu berechnen:
1. Eine Liste der klinischen Fragestellungen 51, die mit dem MR-Scanner beantwortet werden können.
2. Meta-Daten zu diesen klinischen Fragestellungen 51, insbesondere zeit-bezogene Daten, wie z.B. die Scanzeit 52 und/oder typische Vorbereitungszeiten zur Vorbereitung eines Scans.
3. Downtime-Daten, die indizieren, wann das MR-Gerät MR nicht verfügbar ist (z.B. im Rahmen einer Wartung oder eines Services des Gerätes) oder anderweitige Stillstandzeiten.

Ein wesentlicher Vorteil des hier vorgestellten Ansatzes ist es, dass auf dem MR-System MR eine standardisierte Datei, nämlich die Aggregationsdatei A, zur Verfügung gestellt wird, in der die technischen Fähigkeiten des MR Systems abgebildet sind. Diese Datei kann z.B. über eine Web-Schnittstelle (bspw. IP-Adresse) angesteuert werden (unter Berücksichtigung potentieller Sicherheitsanforderungen) und stellt dann für Drittapplikationen die Informationen zu den Scannerfähigkeiten bereit. Die Scannerfähigkeiten können mitunter in der mit dem Scanner beantwortbaren klinischen Fragestellung abgebildet sein, d.h. mit Angaben, welche anatomische Körperregion untersucht werden soll und welche Diagnose fraglich ist (z.B. Verdacht auf Schlaganfall, Tumor, MS, Alzheimer etc.). Weitere Daten können von außen von einem Anwender erfragt werden, z.B. die Atemanhaltezeit des Patienten, das Gewicht, die Größe etc. Es kann umgekehrt aber auch - ausgehend von den Fähigkeiten des Scanners - in der Aggregationsdatei A gespeichert sein, wie lange die Atemanhaltezeit des Patienten sein muss, damit der auf dem Scanner mit den verfügbaren Protokollen untersucht werden kann.

Die Zeit-bezogenen Daten können insbesondere für ein Scheduling verwendet werden, um die zukünftigen Untersuchungen auf dem Gerät zu planen.

Die Aggregationsdatei A kann um eine vollwertige Kalenderfunktion mit entsprechender Schnittstelle nach außen erweitert werden. Diese Ausführungsform der Erfindung ermöglicht es, die oben erwähnten Schedulinginformationen an den Scanner MR zurückzuspielen, sodass der Scanner seine Verfügbarkeit detaillierter zur Verfügung stellen kann. Darüber hinaus kann die Aggregationsdatei A vom KIS/RIS-System proaktiv angefordert und dort empfangen werden, um beispielsweise dann von terminierten Patienten im Voraus die entsprechenden Daten zu laden und vorzubereiten, um die Performance zu verbessern. So könnten bspw. im Nachtlauf vor dem entsprechenden Tag die relevanten Patienteninformationen der Patienten, die am Folgetag terminiert sind, heruntergeladen werden und der Scanner MR kann bereits die entsprechenden Protokolle und Einstellungen vorbereiten. Dies führt zu Performancevorteilen.

Die Aggregationsdatei A wird vorteilhafterweise in einer einheitlichen, strukturierten Sprache erzeugt, um die Scannerinformationen in einer entsprechend definierten Datei zu speichern. Weiterhin kann auf diese Aggregationsdatei A von externen Instanzen, und z.B. aus dem Intranet des Kunden (bspw. des Krankenhauses) und ggf. sogar per Internet zugegriffen werden.

Abschließend sei darauf hingewiesen, dass die Beschreibung der Erfindung und die Ausführungsbeispiele grundsätzlich nicht einschränkend in Hinblick auf eine bestimmte physikalische Realisierung der Erfindung zu verstehen sind. Alle in Verbindung mit einzelnen Ausführungsformen der Erfindung erläuterten und gezeigten Merkmale können in unterschiedlicher Kombination in dem erfindungsgemäßen Gegenstand vorgesehen sein, um gleichzeitig deren vorteilhafte Wirkungen zu realisieren.

Der Schutzbereich der vorliegenden Erfindung ist durch die nachstehenden Ansprüche gegeben und wird durch die in der Beschreibung erläuterten oder den Figuren gezeigten Merkmale nicht beschränkt.

Für einen Fachmann ist es insbesondere offensichtlich, dass die Erfindung nicht nur für MR-Geräte angewendet werden kann, sondern auch für andere medizintechnische Geräte oder Modalitäten, wie z.B. CT, US. Des Weiteren können die Bauteile des MR-Systems auf mehrere physikalische Produkte verteilt realisiert werden.

## Patentansprüche

1. Verfahren zum Erzeugen einer Aggregationsdatei (A) auf einem MR-Scanner (MR), umfassend folgende Verfahrensschritte:
- Erfassen (S1) von Betriebsparametern (BP) auf dem MR-Scanner (MR);
- Aggregieren und Strukturieren (S3) der erfassten Betriebsparameter (BP) in ein vordefiniertes einheitliches Format zum Erzeugen (S4) der Aggregationsdatei (A) .

2. Verfahren nach Patentanspruch 1, wobei die Betriebsparameter (BP) eine Anzahl der verfügbaren Spulen, einen Typ der verfügbaren Spulen, ausführbare Protokolle, eine Magnetfeldstärke, eine Gradientenfeldstärke, eine Feldhomogenität, eine Kanalzahl und/oder einen Bildbereich umfassen.

3. Verfahren nach einem der vorangehenden Patentansprüche, wobei aus der Aggregationsdatei (A) berechnet wird, welche klinische Fragestellung (51) auf dem MR-Scanner (MR) beantwortet werden kann.

4. Verfahren nach einem der vorangehenden Patentansprüche, wobei aus der Aggregationsdatei (A) eine durchschnittliche Scanzeit (52) auf Basis von ausgeführten Protokollen und Messsequenzen berechnet wird.

5. Verfahren nach einem der vorangehenden Patentansprüche, wobei die Betriebsparameter (BP) zumindest teilweise aus lokalen Gerätedaten berechnet werden.

6. Verfahren nach einem der vorangehenden Patentansprüche, wobei eine Zuordnung zwischen jeweils einer beantwortbaren klinischen Fragestellung (51) und einer Menge von erforderlichen Betriebsparametern (BP) des MR-Scanners (MR) gespeichert ist.

7. Verfahren nach einem der vorangehenden Patentansprüche, wobei Zuordnungen zwischen jeweils einer beantwortbaren klinischen Fragestellung (51) und einer Menge von erforderlichen Betriebsparametern (BP) des MR-Scanners (MR) durch Verfahren des maschinellen Lernens gelernt werden.

8. Verfahren nach einem der vorangehenden Patentansprüche, wobei der Schritt des Aggregierens und Strukturierens (S3) ein Konvertieren der erfassten Betriebsparameter (BP) umfasst.

9. Verfahren nach einem der vorangehenden Patentansprüche, wobei die Betriebsparameter (BP) unter Verwendung von Sensoren (S) ermittelt und/oder aus elektronischen Daten berechnet werden.

10. Verfahren nach einem der vorangehenden Patentansprüche, wobei die Aggregationsdatei (A) lokal auf dem MR-Scanner (MR) gespeichert wird und/oder an externe Instanzen übertragbar ist.

11. MR-basiertes System mit zumindest einem MR-Scanner (MR) mit einer elektronischen Instanz (C), die eine Ausführungsumgebung umfasst, in der zumindest eine Anwendung läuft, die zum Erzeugen einer Aggregationsdatei (A) ausgebildet ist, die nach einem Verfahren nach einem der vorangehenden Verfahrensansprüche erzeugt worden ist.

12. Computerprogrammprodukt, das auf einem computerlesbaren Medium gespeichert ist und computerlesbare Programmittel umfasst, die einen Computer veranlassen, die Ausführung des Verfahrens zum Erzeugen einer Aggregationsdatei (A) nach einem der vorangehenden Verfahrensansprüche zu steuern.

13. Computerlesbares Medium, auf dem eine Aggregationsdatei (A) gespeichert ist, die nach einem Verfahren nach einem der vorangehenden Verfahrensansprüche erzeugt worden ist.
